(19) **Europäisches Patentamt**

**European Patent Office**

**Office européen des brevets**

(11) **EP 1 662 665 A1**

(12) **EUROPEAN PATENT APPLICATION**

(43) Date of publication:
**31.05.2006 Bulletin 2006/22**

(51) Int Cl.:
**H03M 3/02** (2006.01)

(21) Application number: **05255261.9**

(22) Date of filing: **26.08.2005**

| | |
|---|---|
| (84) Designated Contracting States:<br>**AT BE BG CH CY CZ DE DK EE ES FI FR GB GR HU IE IS IT LI LT LU LV MC NL PL PT RO SE SI SK TR**<br>Designated Extension States:<br>**AL BA HR MK YU** | (72) Inventor: **Hickling, Ronald M.**<br>**Newbury Park, CA 91320 (US)** |
| (30) Priority: **26.11.2004 US 998212** | (74) Representative: **Hitching, Peter Matthew**<br>**HASELTINE LAKE**<br>**Imperial House**<br>**15-19 Kingsway**<br>**London WC2B 6UD (GB)** |
| (71) Applicant: **Technoconcepts, Inc.**<br>**Van Nuys, CA 91411 (US)** | |

(54) **Direct conversion delta-sigma transmitter**

(57)     A flexible and programmable circuit for generating a radio frequency signal for transmission includes two delta-sigma modulators (62,64), a quadrature clock generator (70) for generating two clock signals (71, 73) having a 90 degree phase difference, two commutators (66, 68) for multiplying the two modulator outputs by +1 and -1 on alternating half cycles of the two quadrature clock signals respectively, a summer (80) for summing the two commutated outputs, and a filter (82) for removing unwanted frequency components before transmission. The circuit directly generates a radio frequency signal without the need for additional frequency translation after the commutation stage.

FIG. 6

**Description**

This invention relates to the field of wireless communication. More particularly, this invention relates to the field of a direct conversion delta-sigma transmitter.

A continuing challenge in the transmission of wireless signals is the process of modulating a carrier signal, i.e., varying the amplitude and/or phase (frequency) in such a way as to convey information onto that carrier, in a mathematically ideal way without producing noise or distortion. Prior art systems have used either analog circuitry exclusively, or have used digital circuitry to generate a baseband or intermediate frequency (IF) waveform followed by conventional analog circuitry to translate the signal up to the required carrier frequency. In either case, the use of conventional analog circuitry has resulted in the generation of spurious signals due to the inability of analog circuitry to accomplish mathematically ideal operations.

U.S. Patent No. 6,748,025, assigned to the assignee of the present invention, discloses the use of commutation in combination with delta-sigma conversion in the architecture of an A/D converter based linear receiver.

Embodiments of the present invention advantageously use a delta-sigma modulator in combination with a commutator to realize an ultra-linear transmitter as well. Direction conversion of a signal from a lower frequency such as a baseband frequency to a higher frequency such as an RF frequency may be achieved according to embodiments of the present invention by using at least one delta-sigma modulator in combination with a commutator. The commutator multiplies the signal by +1 and -1 on alternating half-cycles of a clock such as an RF carrier frequency clock. The resulting waveform contains both the original waveform upconverted to the carrier frequency, plus side lobes that can be filtered out by a bandpass filter (BPF) whose characteristics need not be precisely controlled. A more general transmitter uses a quadrature approach in which two delta-sigma modulators have outputs that are input into respective commutators. The two commutators are clocked 90 degrees apart such that the resulting commutated waveforms are 90 degrees out of phase with each other, i.e., in quadrature, to produce I and Q components. The I and Q components can then be combined. By controlling the generation of the two input waveforms, commutating the two waveforms in quadrature to produce I and Q components and then combining those components, the amplitude and phase and hence frequency of the resulting signal can be arbitrarily controlled. That is, any modulation scheme can be implemented. Because the transmitter circuit does not depend on any precision analog components or filters, the transmitter design is capable of producing virtually any arbitrary waveform over a wide range of frequencies.

When the clock signal is in the radio frequency range, the resulting output is a radio frequency signal which represents the baseband signal upconverted directly to radio frequency without the need for any additional upconversion. Thus, the transmitter eliminates the need for an intermediate frequency conversion step commonly used in radio transmitters.

Additionally, the resulting transmitter can transmit over a wide frequency range merely by changing the clock frequency. If the clock frequency is changed significantly, then the stop band of the BPF will also need to be moved, but moving the stop band of a BPF whose transition bands and other characteristics need not be precisely controlled is a relatively simple task.

Because the invention allows generation of an arbitrary transmitted waveform at an arbitrary carrier frequency merely by programming the device, the present invention achieves a highly flexible and programmable radio frequency transmitter. By reprogramming the device on the fly, the transmitter can be reprogrammed to change the modulation scheme and the carrier frequency essentially instantaneously.

According to a first aspect of the present invention, there is preferably provided a radio frequency transmitter that includes first and second delta-sigma modulators for generating first and second information signals, respectively, clock generation circuitry for generating first and second clocks in the radio frequency range and having a predefined phase difference of 90 degrees, first and second commutators for receiving the information signals and switching the information signals according to the first and second clocks to produce I and Q components of a complex signal, and a summer for combining the first and second commutated signals to produce a combined signal to be transmitted across a wireless network. A controller preferably controls the generation of the first and second information signals such that the resulting transmitted waveform carriers the desired data using the desired modulation scheme.

According to a second aspect of the present invention, there is preferably provided a method of generating a signal for transmission, the method including the steps of generating first and second data words and converting those data words into binary pulses whose pulse widths correspond to the value of the data words respectively, inverting first and second quadrature phase clocks when the first and second binary pulses are present respectively, and not inverting the first and second clocks when the first and second binary pulses are not present respectively, to produce I and Q components, summing the I and Q components, and filtering the summed signal to remove unwanted frequencies, to produce a combined radio frequency transmission signal.

In a further aspect, there is preferably provided a method of generating a signal comprising: providing first and second information signals; multiplying each of the first and second information signals alternatingly by respective +1 and -1 mulitiplicand waveforms, where the +1 and -1 multiplicand waveform for the first signal has approximately a 90 degree phase difference from the +1 and -1 mulitiplicand waveform for the second signal, to produce I and Q quadrature signals; and summing the I and Q quadrature signals to produce a radio frequency signal.

In yet another aspect, there is preferably provided a transmitter having a delta-sigma modulator for receiving a digital input and for generating a baseband signal in response thereto, a commutator for multiplying the baseband signal alternately by a positive factor and a negative factor thereby commutating the signal to produce a second signal, and a filter for removing from the second signal radio frequency components that are significantly outside the range of one half the rate at which the commutation occurs.

In a still further aspect, there is preferably provided a circuit for generating a signal to be transmitted, the circuit comprising: first and second signal generating sections for generating first and second signals, respectively; a first clock switching section for producing a first commutated signal, the first commutated signal being an inverted or a non-inverted version of a first clock according to a state of the first signal; a second clock switching section for producing a second commutated signal, the second commutated signal being an inverted or a non-inverted version of a second clock according to a state of the second signal, the second clock being equal in frequency to the first clock and offset therefrom by a predefined phase difference; and a summing section for adding the first and second commutated signals together to produce a summed signal.

Exemplary embodiments of the invention will be further described below with reference to the drawings, in which like numbers refer to parts having the same or similar function.

FIG. 1A is prior art first order delta-sigma digital-to-analog converter.

FIG. 1B is a prior art second order delta-sigma digital-to-analog converter.

FIG. 1C is a prior art first order delta-sigma analog-to-digital converter.

FIG. 2 is a spectral plot showing the spectrum of a commutated input waveform, where the baseband input to the commutator is centered around $\omega = 0$ and the output is centered around $\omega = \pi$.

FIG. 3 illustrates the output of a delta-sigma modulator for a single bit converter and for a multibit converter.

FIG. 4A is a frequency domain plot showing baseband delta-sigma modulation.

FIG. 4B is a frequency domain plot showing the output spectrum after the baseband delta-sigma modulated signal has been commutated.

FIG. 5 is a constellation plot that represents the discrete states between which the actual output amplitude and phase are interpolated.

FIG. 6 is a block diagram of a delta-sigma transmitter according to a preferred embodiment of the present invention.

FIG. 7 illustrates the use of an XOR gate to implement commutation of a one-bit converter.

FIG. 8 is a schematic diagram of a commutator circuit for converting an input that is coded thermometrically.

FIG. 9 is a schematic diagram of a first implementation of a quadrature clock generator using two "D" flip-flops.

FIG. 10 is a schematic diagram of a second implementation of a quadrature clock generator using a four stage ring oscillator.

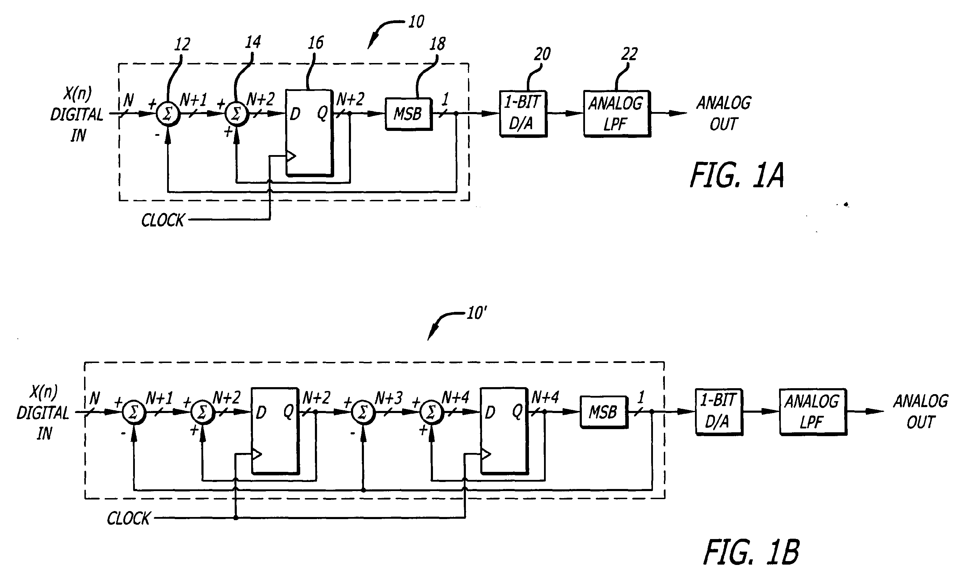

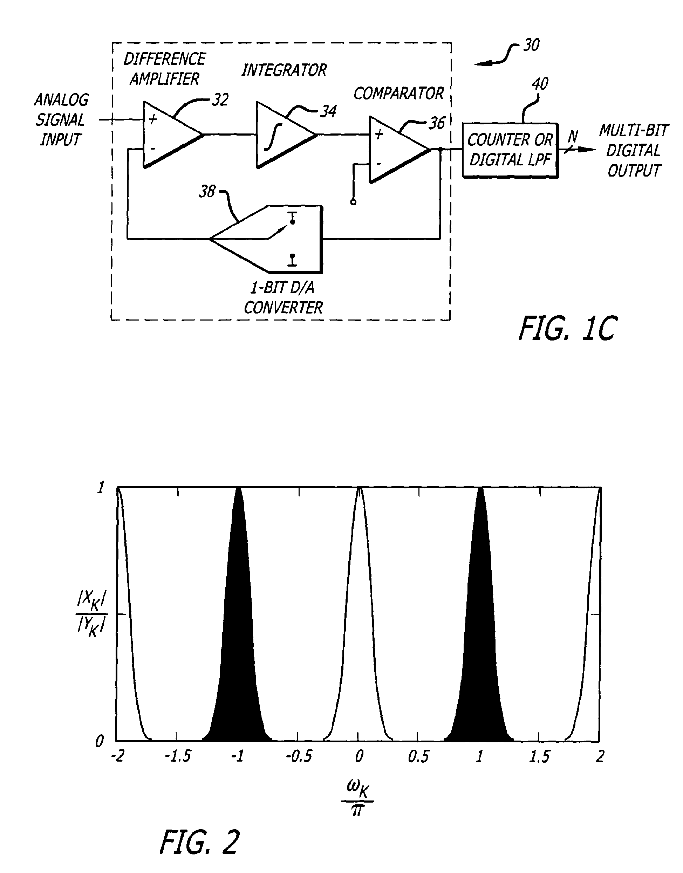

FIG. 1 is block diagram of a conventional delta-sigma digital-to-analog (D/A) converter (DAC). The delta-sigma modulator 10 forms the core of the delta-sigma D/A converter. The basic digital implementation for a first order delta-sigma modulator 10 includes digital difference block 12, digital summer 14, "D" flip-flop or register16, and block 18 which strips off the most significant bit (MSB) from the output of register 16. The name delta-sigma derives from the combination of the difference block 12 (delta) and the summer 14 (sigma). The output of the delta-sigma converter 10 is a pulse train whose duty cycle is proportional to $V_{IN}/V_{MAx}$. That signal is a one-bit (or multibit in the case of a multibit modulator) pulse width modulation (PWM) sequence whose quantization noise is concentrated outside the band of interest.

By converting the PWM output of the delta-sigma converter 10 to analog via 1-bit D/A converter 20 and then low pass filtering the analog signal via analog low pass filter (LPF) 22, the resulting output represents the magnitude of $X_{IN}/X_{mAX}$. When LPF 22 suitably suppresses the outband noise, the resulting analog waveform can have an effective resolution that is extremely high, e.g., greater than 22 bits.

**FIG. 1B** is a conventional second-order delta-sigma D/A converter. Second and higher order delta-sigma converters are also well known and are described in the literature.

**FIG. 1C** is a conventional first order delta-sigma A/D converter. The delta-sigma modulator 30 which constitutes the core of the A/D converter is implemented in the analog domain using a difference amplifier or comparator 32, a continuous time integrator 34, a comparator 36, and 1-bit D/A converter 38. As with the output of delta-sigma modulator 10 in **FIG. 1A,** the output of the delta-sigma converter 30 in **FIG. 1C** is a PWM signal. The PWM signal is converted into a conventional multibit binary word of length N by counter or digital low pass filter 40. A digital low pass filter suppresses the high frequency quantization noise.

Conventional delta-sigma D/A converter technology is capable of clock rates on the order of 10 MHz. Such clock rates are suitable for achieving high resolution output waveforms at audio rates, i.e., significantly lower than 50 kHz. High speed semiconductor technologies such as gallium arsenide (GaAs) and silicon germanium (SiGe) are capable of extending clock rates beyond the 4 GHz realm. However, such clock rates can only enable the generation of waveforms having 5 - 50 MHz of bandwidth, which is clearly insufficient to render a high speed modulated carrier waveform whose center frequency exceeds 2 GHz.

Commutation has been demonstrated to be an effective way of performing frequency translation without the use of an explicit multiplication operation, which for A/D converters cannot be performed distortion-free and for D/A converters requires the use of large amounts of digital circuitry. For example, the multiplication of two 16-bit words generates an output that is 32 bits wide. Furthermore, if explicit multiplication is used, the resulting parallel output would need to be driven into a GHz rate high resolution flash D/A converter, which although easier to design than an A/D converter, is still nevertheless difficult to implement in current generation semiconductor technologies.

Commutation has been previously shown to be an effective method of downconverting a signal as described in copending U.S. patent application serial no. 10/858,611, which is a continuation of serial no. 09/241,994, now U.S. Patent No. 6,748,025, which are hereby incorporated by reference.

With respect to what commutation does to the spectrum of an incoming baseband or otherwise low frequency signal, we note that in discrete-time commutation where a signal x(n) is multiplied by the sequence $(-1)^n$ where n is an integer:

$$y(n) = (-1)^n \cdot x(n)$$

$$Y(e^{j\omega}) = X(e^{j\omega}) \otimes \mathsf{F}\left\{(-1)^n\right\}$$

$$Y(e^{j\omega}) = X(e^{j\omega}) \otimes \frac{1}{2}\left[\sum_{k=0}^{\infty}(1 - e^{-j\pi \cdot k}) \cdot \delta(\omega - \pi \cdot k)\right]$$

$$Y(e^{j\omega}) = \sum_{k \text{ odd}} X(e^{j(\omega - \pi \cdot k)})$$

The above result suggests that an input spectrum centered around $\omega = \Delta$ will produce an output spectrum with images around $\omega = \pi + \Delta, 3\pi + \Delta, 5\pi + \Delta,....$ Thus, an image centered around one half of the clock frequency results, with the nearest repetition of that image being centered around one and one-half times the clock frequency. This is effectively a translation of the spectrum by an amount $\omega = \pi$. Furthermore, aliasing is avoided so long as the bandwidth of the spectrum is limited to $\Delta\omega < \pi/2$.

**FIG. 2** illustrates the effect of commutation in discrete time in the frequency domain. A representative baseband signal (centered around $\omega = 0$) is shown as a line. The effect of commutation in discrete time is shown as a shaded spectrum. Commutation translates a baseband signal to a frequency equal to half the sample rate ($\omega = \pi$) with repetitions at odd harmonics of this frequency.

The basic concept behind a single-bit delta-sigma modulator is that any static voltage $V_{out}$ (or time varying voltage at a suitably low frequency) with a value between two limits $V_{min} < V_{out} < V_{max}$ can be conveyed by the long-term average of a pulse-width modulated (PWM) waveform that switches between $V_{min}$ and $V_{max}$ with a duty cycle $\rho$

such that:

$$V_{out} = \frac{N_{max} \cdot V_{max} + N_{min} \cdot V_{min}}{N_{max} + N_{min}} = \frac{N_{max}}{N_{max} + N_{min}} V_{max} + \frac{N_{min}}{N_{max} + N_{min}} V_{min} = \rho \cdot V_{max} + (1 - \rho) \cdot V_{min}$$

where the quantities $N_{min}$ and $N_{max}$ denote the number of sample periods during which the output of the PWM waveform is held at $V_{min}$ and $V_{max}$, respectively. Needless to say, the equation correctly predicts that as the number of samples used to convey an output value (i.e., $N_{min} + N_{max}$) grows, the precision with which the value of $V_{out}$ can be conveyed improves.

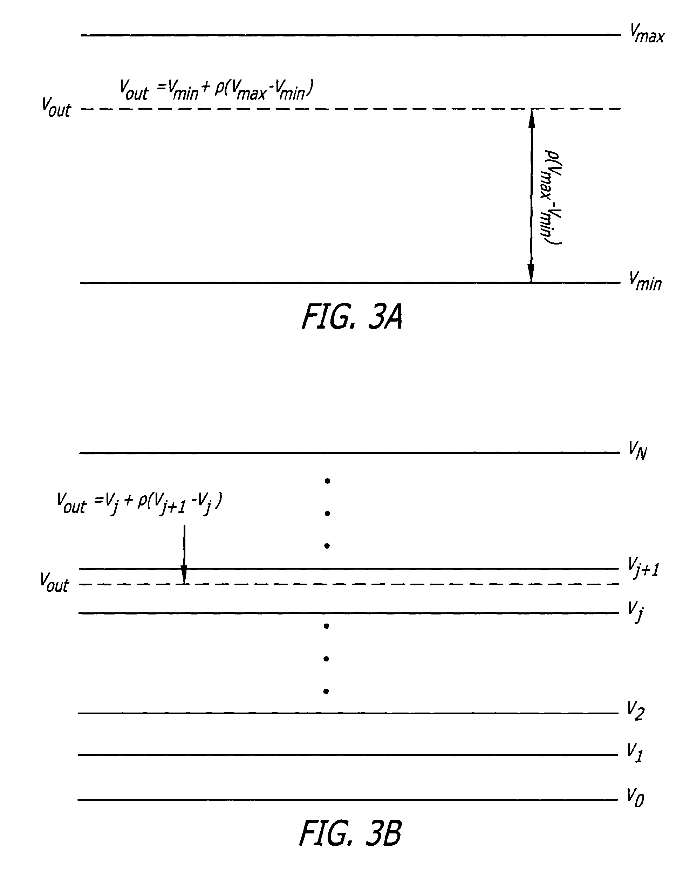

This principle also extends to multibit (multilevel) modulators used as part of delta-sigma modulation loops. Theoretically, delta-sigma converters employing multibit modulators can achieve better resolution than their one-bit counterparts because each sample produced by the modulator has greater resolution. However, realizing this improved resolution is difficult in practice because it is far easier to build a one-bit modulator with, say, 16 bits of precision (i.e., the output voltage is precise to within 0.0015%) than to build a multibit modulator with this same precision. **FIG. 3** compares the operational dynamics of one-bit and multibit delta-sigma modulators when conveying the value of $V_{OUT}$ using a delta-sigma modulator. As illustrated in **FIG. 3A,** using a one-bit modulator a large number of samples must be used to convey $V_{OUT}$ since the "gray levels" between $V_{MIN}$, and $V_{MAX}$ can only be resolved by the duty cycle of a PWM waveform. However, the precision of a single-bit modulator is very high. As illustrated in **FIG. 3B,** using a multibit modulator less mathematical processing is required since the value of $V_{OUT}$ need only be resolved to "gray level" between $V_j$ and $V_{j+1}$. However, barring the use of pre-distortion compensating circuitry, multibit modulators require each reference to have the same precision as the overall modulator. This is difficult to achieve in practice.

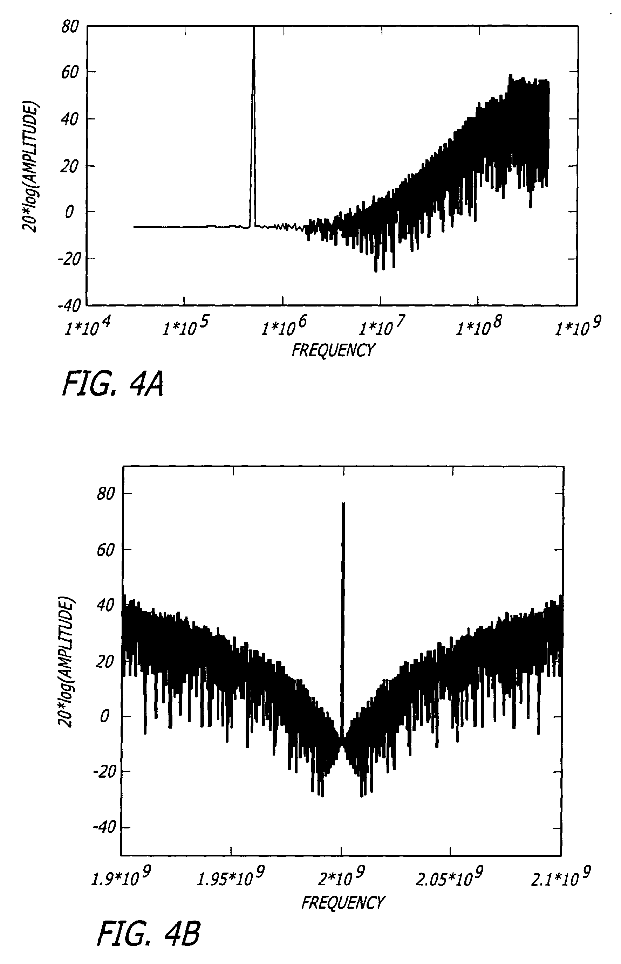

The analysis above has already shown that if a baseband signal conveyed by a delta-sigma modulator is commutated using a sequence $(-1)^n$, then the spectrum of the baseband signal is translated upward in frequency resulting in a spectrum that is centered around one-half of the clock frequency. It follows, then, that if the output waveform of a delta-sigma modulator is upconverted through commutation, then the spectrum of this upconverted waveform will closely approximate that of the desired (ideal) analog waveform at frequencies close to the carrier frequency (half of the clock rate) and would deviate from the desired spectrum at frequencies far removed from the carrier frequency. The simulation in **FIG. 4** shows this behavior. **FIG. 4** is a frequency domain plot of a representations of a sine wave modulating a 2 GHz carrier using delta-sigma modulation and commutation. **FIG. 4A** shows the spectrum of a sine wave produced by a second order delta-sigma modulator. The quantization noise for the modulator is significantly removed from the frequency of the sine wave. The output spectrum contains a rising quantization noise floor. **FIG. 4B** illustrates the result of commutating the baseband signal with a $(-1)^n$ waveform at a clock rate of 4 GHz. The spectrum is now centered around 2 GHz with a low noise floor in the neighborhood of the carrier frequency. Although obscured by the resolution of the plot, there are two separate sidebands, which is the expected result of double sideband suppressed-carrier modulation.

Given that we have just demonstrated that a PWM waveform generated by a delta-sigma modulator can be driven into a commutator to form a signal whose spectral characteristic in the neighborhood of the carrier frequency closely matches that which would be produced by an ideal mixer, it then follows that two such signals can be added in quadrature to form a generalized complex modulation envelope. That is, we can independently vary the amplitude and phase and hence frequency of a carrier signal. This facilitates the generation of any arbitrary modulated carrier waveform since all modulation schemes involve manipulating the instantaneous amplitude and phase of a carrier.

In a sense, commutation is somewhat similar to biphase shift keying (BPSK) and multibit commutation is similar to quadrature amplitude modulation (QAM). Thus, a commutating delta-sigma converter may be regarded as rendering a sine wave (represented as a point on the complex plane) with a prescribed amplitude and phase by alternating back and forth between its two closest neighbors on a QAM (or QPSK for the single bit case) constellation.

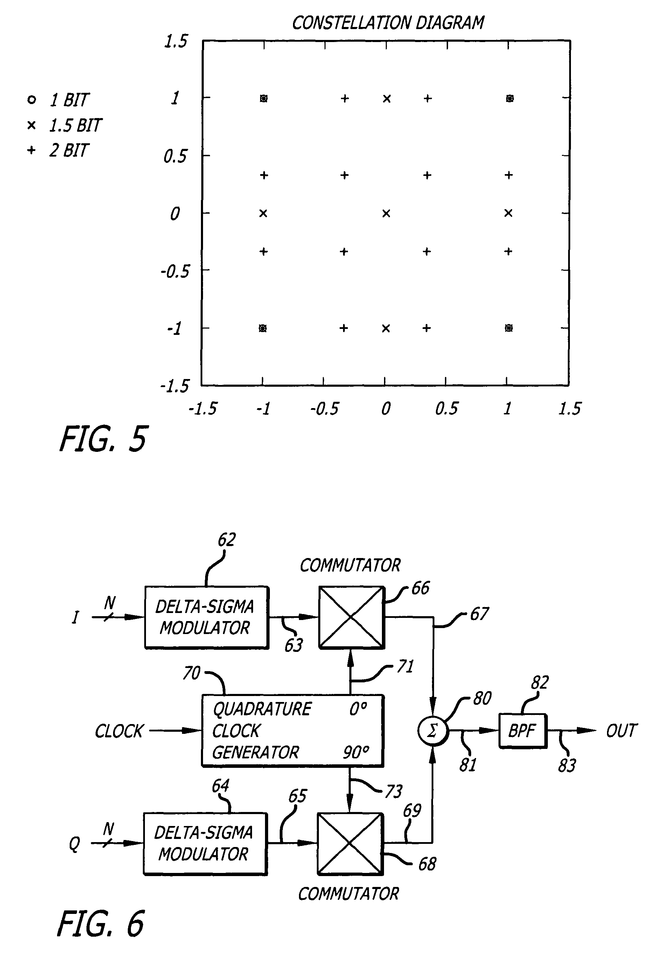

FIG. 5 is the constellation plot that represents the discrete states between which the actual output amplitude and phase are interpolated. Each of the constellations is normalized to a full-scale $\pm$ 1 for the real and imaginary parts, I and Q. A sine wave with a prescribed phase and amplitude would be represented by a vector between the origin of this plot and any point on the plane, with the length of the vector representing its amplitude and the angle between the positive x-axis and the vector representing its phase. The circles represent the constellation produced using single-bit modulators for I and Q. The "X" symbols represent that of using 1.5 bit modulators, i.e., three levels each for I and Q. The "+" symbols represent the constellation associated with using 2-bit D/A converters, i.e., four levels each for I and Q.

A complete delta-sigma transmitter according to one embodiment of the present invention thus consists of a quadrature clock generator, two delta-sigma modulators (one each for I and Q), a pair of commutators (one for each delta-sigma modulator), a summing amplifier, and a bandpass filter. A block diagram of the delta-sigma transmitter is shown in **FIG. 6.** The delta-sigma modulators 62 and 64 each receive N-bit digital inputs, which will be referred to generally herein as N-bit words regardless of the length of those words, and convert those N-bit digital words into pulse width modulated signals 63 and 65, for which the width of the pulse corresponds to the value of the N-bit digital input word. PWM signals 63 and 65 therefore contain one form of the information to be transmitted and can therefore be referred to as information signals. Quadrature clock generator 70 produces two clocks, 71 and 73, which are ideally exactly one quarter cycle, i.e., 90 degrees, out of phase. The commutators digitally multiply the respective PWM signals with the corresponding clock signals. The commutated signals 67 and 69, which represent I and Q components of the complex RF signal to be transmitted, are then combined at summer 80 to produce combined signal 81. Combined signal 81 is then filtered by BPF 82 to produce output signal 83 which typically would be sent to an amplifier (not shown) and then to a transmit antenna (not shown).

The passband of BFP 82 is ideally centered around half the clock frequency, with a passband wide enough to pass the two sidebands each of which have a width equal to the baseband signal. The stop bands need only substantially block the lobes that are produced as a result of the commutation at the clock frequency. As discussed the first lobe to be filtered out will be at a frequency of approximately one and a half times the frequency of the commutation clock signals. The design of band pass filters is well known within the art of filter design. The passband need not be centered exactly at one half the clock frequency. It is only necessary that BPF substantially pass half the clock frequency and the two sidebands produced by the baseband signal, and substantially stop the lobes centered around one and a half times the clock frequency and higher orders.

For radio frequency transmission the clock frequency will also be in the radio frequency range. Thus, the clock frequency may be greater than 1 MHZ, greater than 10 MHz, and greater than 1 GHz. This results in direct conversion of a baseband signal to radio frequency without the need for first converting to an intermediate frequency and then upconverting the intermediate frequency signal to radio frequency.

A controller (not shown) provides the stream of digital inputs to the delta-sigma modulators so that the resulting combined complex waveform represents the information to be transmitted according to the desired modulation scheme. Such a controller may be or may include a microprocessor, a digital signal processor, or other dedicated processor for generating the necessary data stream. Theory and certain details regarding delta-sigma modulators in general can be found in *Delta-Sigma Data Converters: Theory, Design, and Simulation* by Steven R. Norsworthy, Richard Schreier, and Gabor C. Temes (editors) (IEEE Press 1997). Theory and details regarding modulating carrier signals can be found in *Modulation, Detection, and Coding* by Tommy Oberg (Wiley 2001) and *Electronic Communication Systems: Fundamentals Through Advanced* by Wayne Tomasi (Pearson Education 2000) Using these references the practitioner skilled in the relevant art could generate the necessary PWM waveforms for synthesizing the desired complex output signal. In order to offload the processor, the data streams to the delta-sigma modulators could also be provided in part from values stored in sequential access memory (SAM) or randomly accessed access memory such as RAM or ROM accessed via direct memory access (DMA). The design of RF amplifiers and antennas suitable for use in the present application in order to physically transmit the signal produced is also well known.

It will be understood that the multiplication step need not implement multiplication by exactly +1 and -1, as the presence of a fixed amplification or attenuation at any stage in the process does not affect the general operation of the circuit. Thus, as used herein, the terminology of multiplying by +1 or -1 refers generally to multiplying by any positive and negative factor. Similarly, the summing steps and filtering steps, and other steps along the way, can also include amplification or attenuation by some factor without substantially affecting the analysis.

**Commutator Design**

The commutator is a block that multiplies the input by +1 and -1 alternately during opposite half-cycles of the incoming clock signal. More generally, the commutator switches the incoming information signal according to the current state (HI or LO) of the clock signal. For analog signals, the commutator is realized by using a commutating amplifier implemented as a complementary output amplifier that alternates its output terminals during opposite half-cycles of the clock signal. For digital circuits, the concept of a commutator must take on a somewhat different realization. Although many implementation of a commutator are possible and will be apparent to those of ordinary skill in the art, the following addresses two possible implementations.

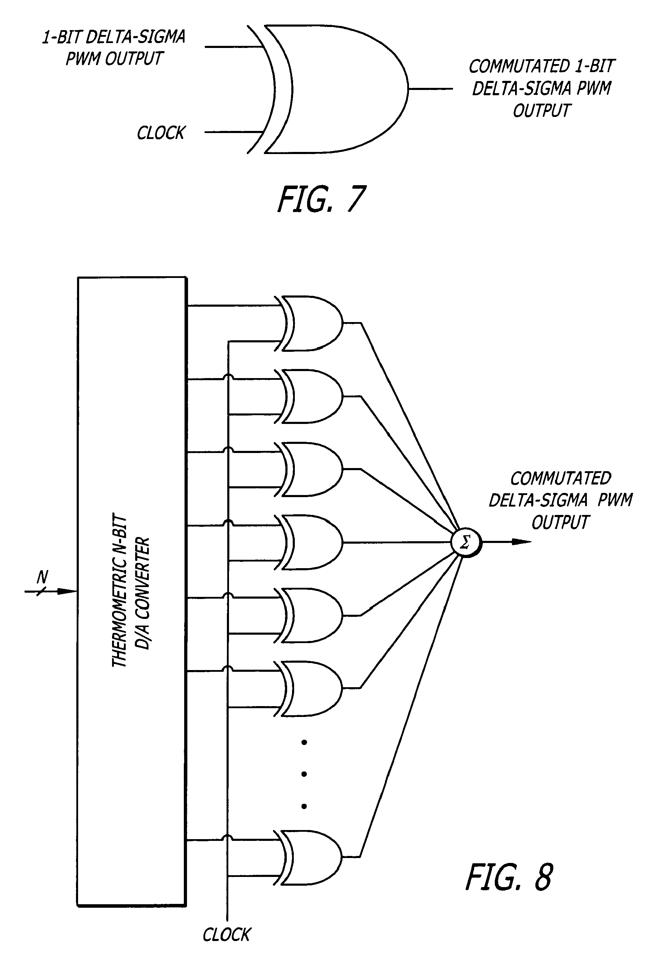

A simple one-bit commutator is implemented using an exclusive-or (XOR) gate as shown in **FIG. 7.** The commutator can be thought of as either inverting the clock signal according to the HI or LO state of the PWM output from the delta-sigma modulator, or it can be thought of as inverting the PWM output signal from the delta-sigma modulator according to the HI or LO state of the commutation clock.

For a thermometric converter (i.e., one in which an N-bit word is represented by $2^N-1$ lines and in which an integer No is represented by No of those lines carrying a logic "1" and $2^N-1 -N_o$ of those lines carrying a logic "0") a commutator can be implemented by driving an XOR gate with each of the $2^N-1$ output lines and summing the result. **FIG. 8** illustrates a commutator circuit for a converter that is coded thermometrically.

**Quadrature Clock Generator Design**

A variety of methods are available for developing a pair of clock signals that are 90° out of phase with one another as will be apparent to those of skill in the art. Two such methods, both appropriate for implementation on a semiconductor integrated circuit, are elaborated here for illustration.

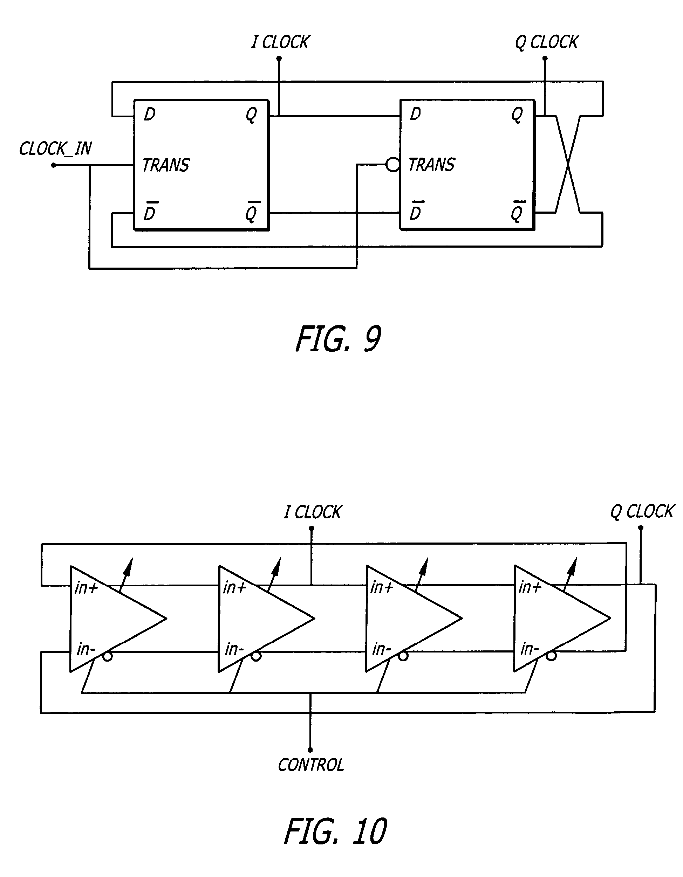

**FIG. 9** is a diagram which shows a first implementation of a quadrature clock generator using two "D" flip-flops. This implementation uses a master and slave outputs of a divide-by-2 circuit, which is driven by a double frequency clock. Since the master and slave latches are activated on opposite half-phases of the clock, the effective phase shift is one quarter of a period, i.e., 90 degrees.

**FIG. 10** illustrates a second implementation of a quadrature clock generator using a four stage ring oscillator. In this implementation a voltage-controlled ring oscillator is locked to a stable external clock using a phaselocked loop (PLL). Provided this ring oscillator is configured to have an even number of stages, a stable frequency source that produces both "in phase" and "quadrature" output may be implemented.

It will be understood that although a 90 degree phase difference for the quadrature clocks is ideal, a quadrature clock generator which generates clocks whose phase difference deviates only slightly from the ideal 90 degree predefined phase difference will still operate adequately within the transmitter of the present invention under most circumstances, so the phase difference need only be approximately 90 degrees or one quarter cycle.

The present invention allows a flexible, highly programmable transmitter to be implemented that does not require careful tuning or control of components. Using the present invention, a transmitter can be implemented that is capable of transmitting on a carrier frequency that can change by a least a factor of two under software control. The present invention could be implemented in nearly any wireless application, and is well suited for mobile cellular telephones and other spread spectrum applications including frequency hopping spread spectrum.

The present invention could also be used to transmit analog signals. By commutating an analog output such as the output of a one-bit delta-sigma D/A converter, or two delta-sigma D/A converters for the full quadrature case, the present invention allows a flexible analog signal transmitter to be constructed as well.

It will be appreciated that the term "present invention" as used herein should not be construed to mean that only a single invention having a single essential element or group of elements is presented. Similarly, it will also be appreciated that the term "present invention" encompasses a number of separate innovations which can each be considered separate inventions. Although the present invention has thus been described in detail with regard to the preferred embodiments and drawings thereof, it should be apparent to those skilled in the art that various adaptations and modifications of the present invention may be accomplished without departing from the spirit and the scope of the invention. Accordingly, it is to be understood that the detailed description and the accompanying drawings as set forth hereinabove are not intended to limit the breadth of the present invention, which should be inferred only from the following claims and their appropriately construed legal equivalents.

**Claims**

1. A radio frequency transmitter comprising:

   first and second delta-sigma modulators for generating first and second information signals, respectively;
   clock generation circuitry for generating first and second clock signals, said first and second clock signals each being greater than 1 MHZ and having a predefined relative phase difference;
   a first commutator for receiving the first information signal and the first clock signal, and for switching the first information signal according to the first clock signal to produce a first commutated signal;
   a second commutator for receiving the second information signal and the second clock signal, and for switching the second information signal according to the second clock to produce a second commutated signal; and
   a summing circuit for combining the first and second commutated signals to produce a combined signal to be transmitted across a wireless network.

2. The transmitter of claim 1 further comprising at least one band pass filter having a passband centered at approximately one half the frequency of the first and second clocks for removing unwanted frequency components from the combined signal before the combined signal is transmitted.

3. The transmitter of claim 1 wherein the predefined relative phase difference is approximately 90 degrees.

4. The transmitter of claim 1 further comprising:

a controller for generating the first and second information signals such that the combined signal represents a radio frequency signal that is controlled in both amplitude and phase according to the first and second information signals.

**5.** The transmitter of claim 4 wherein the radio frequency signal is a spread spectrum signal.

**6.** The transmitter of claim 4 wherein the transmitter is a mobile telephone transmitter.

**7.** The transmitter of claim 4 wherein:

the clock generation circuitry has a controllable frequency range of at least a factor of two, such that the transmitter is capable of transmitting information over a frequency range of at least a factor of two.

**8.** The transmitter of claim 1 wherein the information signals received by the commutators are pulse width modulated signals.

**9.** A method of generating a signal comprising:

generating a first data word and converting said first data word into a first binary pulse whose pulse width corresponds to a value of said first data word;
generating a second data word and converting said second data word into a second binary pulse whose pulse width corresponds to a value of said second data word;
inverting a first clock signal when said first binary pulse is present, and not inverting said first clock signal when said first binary pulse is not present, to produce a first signal component;
inverting a second clock signal when said second binary pulse is present, and not inverting said second clock signal when said second binary pulse is not present, to produce a second signal component; and
summing said first and second signal components to produce a combined radio frequency transmission signal.

**10.** The method of claim 9 wherein the first and second signal components define I and Q components of a complex radiofrequency signal.

**11.** The method of claim 9 wherein the first and second clock signals have a relative phase difference of approximately one quarter cycle.

**12.** The method of claim 9 further comprising filtering said combined radio frequency transmission signal to remove frequency components which are greater than or equal to one and a half times the frequency of the first clock signal and the second clock signal and transmitting the combined radio frequency transmission signal across a wireless transmission link.

FIG. 1A

FIG. 1B

EP 1 662 665 A1

FIG. 1C

FIG. 2

$V_{max}$

$V_{out}$ $\quad V_{out} = V_{min} + \rho(V_{max} - V_{min})$

$V_{out}$

$\rho(V_{max} - V_{min})$

$V_{min}$

## FIG. 3A

$V_N$

$V_{out} = V_j + \rho(V_{j+1} - V_j)$

$V_{j+1}$

$V_{out}$

$V_j$

$V_2$

$V_1$

$V_0$

## FIG. 3B

FIG. 4A

FIG. 4B

CONSTELLATION DIAGRAM

o  1 BIT
×  1.5 BIT
+  2 BIT

**FIG. 5**

**FIG. 6**

1-BIT DELTA-SIGMA
PWM OUTPUT

CLOCK

COMMUTATED 1-BIT
DELTA-SIGMA PWM
OUTPUT

## FIG. 7

THERMOMETRIC N-BIT
D/A CONVERTER

N

CLOCK

COMMUTATED
DELTA-SIGMA PWM
OUTPUT

$\Sigma$

## FIG. 8

FIG. 9

FIG. 10

**European Patent Office**

**EUROPEAN SEARCH REPORT**

Application Number

EP 05 25 5261

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 6 339 621 B1 (COJOCARU CHRISTIAN ET AL) 15 January 2002 (2002-01-15) * column 3 - column 10; figures 4,6 * ----- | 1-12 | H03M3/02 |
| X | US 6 094 458 A (HELLBERG ET AL) 25 July 2000 (2000-07-25) * column 1 - column 18; figure 14 * ----- | 1-12 | |
| X | EP 1 161 044 A (MATSUSHITA ELECTRIC INDUSTRIAL CO., LTD) 5 December 2001 (2001-12-05) * column 7 - column 18; figure 8 * ----- | 1-12 | |
| X | EP 0 426 560 A (MATRA COMMUNICATION) 8 May 1991 (1991-05-08) * page 3 - page 5; figure 2 * ----- | 1-12 | |

TECHNICAL FIELDS
SEARCHED    (Int.Cl.7)

H03M
H04B

The present search report has been drawn up for all claims

| Place of search | Date of completion of the search | Examiner |
|---|---|---|
| Munich | 2 November 2005 | Morrish, I |

EPO FORM 1503 03.82 (P04C01)

## ANNEX TO THE EUROPEAN SEARCH REPORT
## ON EUROPEAN PATENT APPLICATION NO.

EP 05 25 5261

This annex lists the patent family members relating to the patent documents cited in the above-mentioned European search report.
The members are as contained in the European Patent Office EDP file on
The European Patent Office is in no way liable for these particulars which are merely given for the purpose of information.

02-11-2005

| Patent document cited in search report | | Publication date | Patent family member(s) | | Publication date |
|---|---|---|---|---|---|
| US 6339621 | B1 | 15-01-2002 | CA | 2213156 A1 | 15-02-1999 |
| US 6094458 | A | 25-07-2000 | AU | 740623 B2 | 08-11-2001 |
| | | | AU | 4140297 A | 02-04-1998 |
| | | | CA | 2262511 A1 | 19-03-1998 |
| | | | CN | 1229546 A | 22-09-1999 |
| | | | DE | 69731535 D1 | 16-12-2004 |
| | | | EP | 0914728 A1 | 12-05-1999 |
| | | | JP | 2001503210 T | 06-03-2001 |
| | | | KR | 2000035813 A | 26-06-2000 |
| | | | SE | 507373 C2 | 18-05-1998 |
| | | | SE | 9603256 A | 07-03-1998 |
| | | | WO | 9811683 A1 | 19-03-1998 |
| EP 1161044 | A | 05-12-2001 | US | 2001050962 A1 | 13-12-2001 |
| EP 0426560 | A | 08-05-1991 | AT | 110509 T | 15-09-1994 |
| | | | DE | 69011811 D1 | 29-09-1994 |
| | | | DE | 69011811 T2 | 15-12-1994 |
| | | | DK | 426560 T3 | 19-09-1994 |
| | | | ES | 2060986 T3 | 01-12-1994 |
| | | | FR | 2653959 A1 | 03-05-1991 |
| | | | HK | 1004587 A1 | 27-11-1998 |
| | | | NO | 904749 A | 03-05-1991 |
| | | | PT | 95768 A | 30-06-1992 |

EPO FORM P0459

For more details about this annex : see Official Journal of the European Patent Office, No. 12/82